Europäisches Patentamt

European Patent Office

Office européen des brevets

(19)

(11) Veröffentlichungsnummer: **0 121 115**
**A1**

(12)

# EUROPÄISCHE PATENTANMELDUNG

(21) Anmeldenummer: 84102238.7

(22) Anmeldetag: 02.03.84

(51) Int. Cl.³: **A 61 K 33/42**
A 61 K 33/06, A 61 K 31/52
//(A61K33/42, 31/52, 31/375,
31/07), (A61K33/06, 31/52,
31/375, 31/07), (A61K31/52,
31/375, 31/195, 31/37)

(30) Priorität: 02.03.83 DE 3307382

(43) Veröffentlichungstag der Anmeldung:
10.10.84 Patentblatt 84/41

(84) Benannte Vertragsstaaten:
AT CH DE FR GB IT LI NL

(71) Anmelder: Walter, Hans-Peter
Ludwig-Thoma-Strasse 29A
D-8022 Grünwald(DE)

(72) Erfinder: Walter, Hans-Peter
Ludwig-Thoma-Strasse 29A
D-8022 Grünwald(DE)

(74) Vertreter: Hrabal, Ulrich, Dr. Dipl.-Chem. et al,
Patentanwälte Popp, Sajda, v. Bülow, Hrabal & Partner
Widenmayerstrasse 48 Postfach 86 06 24
D-8000 München 86(DE)

(54) Arzneimittel, geeignet zur Behandlung von entzündlichen Veränderungen der Bronchialschleimhaut.

(57) Beschrieben wird ein Arnzneimittel, das pharmakologisch brauchbare Magnesiumverbindungen und Etofyllin neben Vitamin A und Vitamin C enthalten. Es ist besonders geeignet zur Behandlung entzündlicher Veränderungen der Bronchialschleimhaut.

EP 0 121 115 A1

Hans-Peter Walter

8022 Grünwald

M/WAL-11-EP

**0121115**

## Arzneimittel, geeignet zur Behandlung von entzündlichen Veränderungen der Bronchialschleimhaut

Die Erfindung betrifft ein Arzneimittel, das besonders zur Behandlung von entzündlichen Veränderungen der Bronchialschleimhaut geeignet ist.

Es ist bekannt, daß akute Infekte des Bronchialsystems sich grundlegend von chronischen Bronchitiden unterscheiden. Die Ursache der chronischen Bronchitis ist noch nicht genügend geklärt. Deshalb definiert die Weltgesundheitsorganisation die chronische Bronchitis als eine Erkrankung, die mindestens über zwei Jahre und während dieses Zeitraums mindestens drei Monate hindurch und an den meisten Tagen der Woche mit Husten und Auswurf einhergeht.

Es lassen sich drei Formen im Ablauf der Erkrankung voneinander abgrenzen. Zunächst kommt es zu einer vermehrten Schleimproduktion im Bronchialsystem mit produktivem Husten, dann treten häufig rezidivierende bakterielle Superinfekte auf und schließlich entwickeln sich Strömungsbehinderungen in Bronchien und Bronchiolen und es entsteht eine chronische - obstruktive Bronchitis.

Voruntersuchungen haben gezeigt, daß bei entzündlichen Veränderungen des Tracheobronchialsystems ein Ansteigen des Gewebshistamins zu beobachten ist.

Die Möglichkeiten, medikamentös in den Histaminstoffwechsel einzugreifen, sind vielfältig:

a) Sie bestehen einmal mit den bekannten $H_1$- und $H_2$-Rezeptorantagonisten, die jedoch bekanntlich in der Behandlung von akuten bzw. chronischen Lungenerkrankungen, speziell auch beim Asthma bronchiale, wenig Erfolg erzielt haben (Ulmer et al., Reinhardt, Reimann et al., 1982).

b) Zum zweiten in der Gabe von sogenannten Mastzellmembranstabilisatoren, die ihren festen Platz in der Asthma-Therapie gefunden haben (Altunyan, 1981, Cox, Davis, Pepys et al., 1981).

c) Zum dritten durch den Eingriff in den Aufbau des Histamins durch Blockade der Umwandlung von Histidin zu Histamin. Die Langzeitgabe von Histidindecarboxylase-Blockern führt zu einem Abfall des Gewebshistamins auch bei lungengesunden Probanden (Reimann et al., 1982).

In der Behandlung akuter Entzündungen wurden in der Vergangenheit verschiedene Arzneimittel, die in den Histaminstoffwechsel eingreifen, eingesetzt. Dabei hatte es sich jedoch gezeigt, daß durch Blockierung des Histamins am Rezeptor kein Erfolg zu erzielen war.

Aufgabe der Erfindung ist die Bereitstellung eines Arzneimittels zur Behandlung entzündlicher Veränderungen der Bronchialschleimhaut, das zu einer raschen Besserung des Krankheitszustands führt.

Diese Aufgabe wird durch das in den Patentansprüchen beschriebene Arzneimittel gelöst, das den Gegenstand der Erfindung darstellt.

Es hat sich überraschenderweise gezeigt, daß es bei Patienten, denen das erfindungsgemäße Arzneimittel verabreicht wurde, zu einer sehr schnellen Besserung des Allgemeinbefindens und gleichzeitig auch zu einer Reduktion des Gewebshistamins kam.

Das erfindungsgemäße Arzneimittel ist ein Kombinationspräparat, das neben pharmakologisch brauchbaren Magnesiumverbindungen Etofyllin $\sqrt{\ }$7-(2-Hydroxyethyl)-theophyllin$\_7$ sowie Vitamine, insbesondere hohe Dosierungen von Vitamin A und geringere Dosierungen von Vitamin C, neben gegebenenfalls Vitamin E, enthält.

Geeignete Magnesiumverbindungen, die in dem erfindungsgemäßen Präparat eingesetzt werden können, sind übliche, in der Pharmakologie verwendete Magnesiumverbindungen, wie zum Beispiel Magnesium-Salze mit pharmakologisch verträglichen Anionen. Beispiele für spezielle Magnesiumverbindungen sind: Magnesiumhydrogenphosphat, Magnesiumglutimat, Magnesiumascorbat, Magnesium-II-aminosäurechelate, Magnesiumgluconat, Magnesiumchlorid, Magnesiumhydrogenglutamat und Magnesiumorotat. Bevorzugt sind Magnesiumhydrogenphosphat, z. B. als Trihydrat und Magnesium-L-hydrogenglutamat.

Vitamin A (Vitamin $A_1$ und/oder $A_2$) wird zum Beispiel in der Form von Retinolpalmitat zugesetzt. Vitamin E, das als "Antisterilitätsvitamin" bekannte α-Tocopherol, kann z. B. als Acetat vorhanden sein.

Als weitere Hilfssubstanzen kann das erfindungsgemäße Arzneimittel beispielsweise Zinkoxid (tägliche Dosierungsmenge 1-3 mg, z. B. 1,5 oder 2,5 mg), Methionin (tägliche Dosierungsmenge 40-80 mg, z. B. 60 mg) und/oder ein übliches Mucolyticum in einer mucolytisch wirksamen Menge enthalten, z. B. Ambroxol (trans-4-(2-Amino-3,5-dibrombenzylamino)-cyclohexanol als Hydrochlorid; tägliche Dosismenge etwa 50-150 mg, z. B. etwa 100 mg) oder Acetylcystein (N-Acetyl-L-cystein, tägliche Dosismenge etwa 100 bis 300 mg, z. B. etwa 200 mg).

Das erfindungsgemäße Arzneimittel kann bevorzugt zur oralen Verabreichung formuliert werden; beispielsweise zu Tabletten, Dragees, Pulvern, Granulaten oder Kapseln,

0121115

die sämtliche oder in getrennter Form einzelne Bestandteile des Kombinationspräparats enthalten. Die Formulierung erfolgt mit für diese Zwecke üblichen Hilfs- und Trägerstoffen. Man bedient sich auf diesem Gebiet üblicher Formulierungsverfahren. Es kann auch in üblicher Weise zur parenteralen, insbesondere intravenösen Verabreichung formuliert werden. Man bedient sich dazu üblicher Lösungs- bzw. Emulgiermittel. Auch Konzentrate zur Auflösung und/oder Emulgierung unmittelbar vor der Verabreichung sind geeignet.

Die Gewichtsverhältnisse der Einzelkomponenten können im weiten Bereich variieren und sind nicht kritisch. Sie liegen beispielsweise bei 1 bis 4 Gewichtsteilen Etofyllin pro Gewichtsteil Magnesium als Magnesiumionen.

Die Dosierung richtet sich nach dem Patienten und dessen Gesundheitszustand. Die tägliche Magnesiumzufuhr sollte 10 mg/kg Körpergewicht nicht überschreiten; Vitamin A sollte in einer Tagesdosis verabreicht werden, die 50 000 I.E. nicht überschreitet. Als besonders geeignet erwiesen hat sich eine tägliche Dosis von bis zu etwa 22 500 I.E. Vitamin A, zum Beispiel als Retinolpalmitat. Beispielsweise können etwa bis zu 200 mg Retinolpalmitat täglich verabreicht werden. Die tägliche Dosis an Etofyllin in dem erfindungsgemäßen Kombinationspräparat liegt bevorzugt bei etwa 100-200 mg, insbesondere bei bis zu etwa 150 mg. Die tägliche Dosis an Vitamin C liegt bei etwa 50 mg bis über 200 mg.

Ein Kombinationspräparat in Dosiseinheitsform, das beispielsweise zur einmal täglichen Verabreichung geeignet ist, kann bevorzugt enthalten:

Vitamin A:     bis zu 50 000 I.E., insbesondere etwa
               etwa 25 000 I.E., z. B. bis zu etwa 200 mg
               Retinolpalmitat
Etofyllin:     100-200 mg, z. B. etwa 150 mg

Magnesium:      bis zu 10 mg/kg Körpergewicht des zu behandelnden Patienten; also insgesamt bis zu etwa 50-70 mg Magnesium; z. B. 250-300 mg Magnesiumhydrogenphosphat (insbesondere als Trihydrat), plus 250-300 mg Magnesiumglutaminat (z. B. als Magnesium-L-hydrogenglutamat); oder 400-600 mg, insbesondere bis zu etwa 500 mg Magnesiumorotat

Vitamin C:      mindestens etwa 50 mg, bevorzugt 200 mg oder mehr;

Vitamin E:      etwa 30 mg bis etwa 100 mg als α-Tocopherolacetat,

und gegebenenfalls einen oder mehrere der folgenden Bestandteile:

Zinkoxid:       1-3 mg, z. B. 1,5 oder 2,5 mg

Methionin:      40-80 mg, z. B. 60 mg

Mucolyticum:    z.B. Ambroxol.HCl in einer Menge von 50 bis 150 mg, z. B. etwa 100 mg.

Wenn ein Dosiseinheitspräparat zur 2- oder 3mal täglichen Verabreichung formuliert werden soll, so kann es die Hälfte oder ein Drittel der vorstehend angegebenen Menge der Bestandteile enthalten.

Es hat sich gezeigt, daß das erfindungsgemäße Kombinationarzneimittel besonders geeignet zur Behandlung entzündlicher Veränderungen der Bronchialschleimhaut ist. Es kann sich dabei um Veränderungen bzw. Erkrankungen der Bronchialschleimhaut handeln, die bewirkt werden durch Rauchen, berufliche Exposition gegenüber Staub, Dämpfen und Gasen, allgemeine Luftverschmutzung, rezidivierende Infekte, bronchiale Allergien, Klimaeinflüsse und chronisch deformierende Bronchiopatin. Ein Hauptanwendungszweck für das erfindungsgemäße Arzneimittel liegt in der Bekämpfung von durch Nikotin abusus bedingten Erkrankungen der Bronchialschleimhaut, zum Beispiel des sogenannten Raucherhustens. Das erfindungsgemäße Kombinationspräparat hat sich dabei bekannten Histidindecarboxylase-Blockern als überlegen erwiesen. Überraschenderweise wird durch das erfindungsgemäße Kombinationspräparat eine schnellere Besserung des Allgemeinbefindens bei gleichzeitiger Verringerung des Gebeshistamins erzielt.

Die folgenden Beispiele dienen zur Erläuterung der Erfindung.

Beispiel 1 - Formulierung eines Kombinationspräparats zur oralen Verabreichung (als Dragees):

Etofyllin 150 mg

Retinolpalmitat 22 500 I.E.

α-Tocopherolacetat 30 mg

Magnesiumhydrogenphosphat 250 mg

Magnesiumglutaminat 250 mg

Zinkoxid (als üblicher Zusatzstoff) 2,5 mg

Ascorbinsäure 50 mg

Beispiel 2 - Formulierung eines Kombinationspräparats zur oralen
Verabreichung (in der Form von zwei Dragees):

Dragee 1:                                    Dragee 2:
Magnesiumhydrogenphosphat 250 mg             Etofyllin 150 mg
Magnesiumglutaminat 250 mg                   Retinolpalmitat 22 500 I.E.
Ascorbinsäure (Vitamin C) 50 mg              α-Tocopherolacetat 30 mg
Zinkoxid 2,5 mg

Beispiel 3 - Formulierung in der Form von zwei Dragees:

Dragee 1:                                    Dragee 2:
Retinolpalmitat 22 500 I.E.                  Magnesiumhydrogenphosphat 300 mg
α-Tocopherolacetat 30 mg                     Magnesiumglutaminat 300 mg
Etofyllin 150 mg                             Ascorbinsäure 50 mg

Beispiel 4 - Pharmakologische Untersuchung des Kombinationspräparats nach Beispiel 1 und 2:

In die Untersuchung einbezogen wurden freiwillige Probanden
beiderlei Geschlechts, die über einen Zeitraum von mehr als
vier Jahren mehr als 25 Zigaretten täglich inhaliert hatten
und diese Gewohnheit auch während der Versuchsdauer beibehielten.

Als hartes Kriterium einer entzündlichen Veränderung der
Bronchialschleimhaut wurde die makroskopische Betrachtung
der großen Bronchien mittels Fiberbronchoskop herangezogen
(Score-Einteilung 1 - 3 je nach Schweregrad). Die mikroskopische Untersuchung galt nur als Bestätigung.

Doppelblind und randomisiert wurden den Probanden entweder ein HDC-Blocker oder das Kombinationspräparat von Beispiel 2 verabreicht.

Altersverteilung der Gruppen:

Gruppe I  :  HDC-Blocker, 30 Probanden, Durchschnittsalter 27 Jahre (21 - 36 Jahre)

Gruppe II :  erfindungsgemäßes Kombinationspräparat, 30 Probanden, Durchschnittsalter 30 Jahre (26 - 39 Jahre)

Alle Probanden wurden zum Zeitpunkt Null, also vor Einnahme der Medikamente fiberbronchoskopisch untersucht. Jeweils zwei Untersucher befundeten das makroskopische Bild und nahmen die Einteilung in die Entzündungsgrade vor. In allen Fällen wurden mehrere Schleimhautbiopsien an definierten Stellen für die histologische und für die Histamin- und Mastzelluntersuchungen entnommen.

Die Probanden wurden angehalten, über einen Zeitraum von 14 Tagen jeweils morgens und abends die der Gruppeneinteilung entsprechenden Medikamente einzunehmen.

Am Ende der 14 Tage wurde eine erneute Fiberbronchoskopie mit makroskopischer, mikroskopischer und histochemischer Untersuchung durchgeführt.

Das Histamin wurde fluorometrisch nach der Methode von Lorenz et al., 1972, die Mastzellzahl mittels Orthophthaldialdehyd nach der Methode nach Reimann et al., 1980, bestimmt.

Die statistische Auswertung erfolgte mit dem Student-t-Test für gepaarte Daten und mit dem Wilcoxon-Rang-Summen-Test.

ERGEBNISSE:

Alle Probanden zeigten vor Therapiebeginn makroskopisch und histologisch gesicherte entzündliche Veränderungen.

Tabelle 1 zeigt die Aufteilung der entzündlichen Veränderungen vor Behandlung entweder mit einem HDC-Blocker (Gruppe I) oder mit dem erfindungsgemäßen Kombinationspräparat (Gruppe II).

In der gleichen Tabelle ist die Veränderung innerhalb der mikroskopischen und makroskopischen Beurteilung der Schleimhautschäden aufgeführt.

Es kommt zu einer deutlichen Verbesserung der geschädigten Schleimhautverhältnisse, die bei dem erfindungsgemäßen Kombinationspräparat besser ausfällt als nach Gabe des Histidindecarboxylase-Blockers.

Tabelle 2 zeigt die subjektiven Beschwerden, die bei den Probanden vor und nach Therapie vorhanden waren.

Bereits nach 8 Tagen kam es zu einer subjektiven Besserung der angegebenen Beschwerden wie Husten, Auswurf und retrosternalem Brennen; dabei schnitt die Gruppe II besser ab als Gruppe I.

Bereits nach 14 Tagen waren über 90% der Probanden bei Einnahme des erfindungsgemäßen Kombinationspräparats beschwerdefrei, bei Einnahme des HDC-Blockers lag dieser Prozentsatz nur bei etwa 65%.

Tabelle 1 - Makroskopische und histologische Bewertung der Bronchialschleimhaut vor und nach Behandlung

| | HDC-Blocker (I) | | | erfindungsgemäßes Kombinations-präparat (II) | | |
| --- | --- | --- | --- | --- | --- | --- |
| | | vor | nach | | vor | nach |
| Score | | | | Score | | |
| 0 | | | 10 | 0 | | 18 |
| 1 | | 7 | 14 | 1 | 4 | 10 |
| 2 | | 17 | 5 | 2 | 16 | 2 |
| 3 | | 6 | 1 | 3 | 10 | 0 |

Tabelle 2 - Subjektive Beschwerdefreiheit, die bei allen Probanden bei der Anfangsbefragung vorhanden waren: Husten, Auswurf, Kratzen im Hals und retrosternal brennendes Gefühl

| Gruppe | | HDC-Blocker I (%) | erfindungsgemäßes Kombi-nationspräparat II (%) |
| --- | --- | --- | --- |
| | Tage | | |
| | 8 | 50 | 82 |
| | 14 | 64 | 90 |
| | 28 | 72 | 92 |

Das erfindungsgemäße Präparat war gut verträglich, vereinzelt beobachtete Unverträglichkeitsreaktionen waren unerheblich.

Aufgrund der günstigen Wirkung des erfindungsgemäßen Kombinationspräparates ergibt sich die Indikation für eine Langzeitbehandlung von Patienten mit akuten und chronischen Bronchialschleimhautveränderungen.

Hans-Peter Walter                    M/WAL-11-EP
8022 Grünwald

P a t e n t a n s p r ü c h e
für die Vertragstaaten DE, NL, IT, FR, GB, CH, LI

1. Arzneimittel mit einem Gehalt an Etofyllin, Vitamin A, einer oder mehreren pharmakologisch brauchbaren Magnesiumverbindungen und Vitamin C, gegebenenfalls neben üblichen Hilfs- und Trägerstoffen.

2. Arzneimittel nach Anspruch 1, dadurch gekennzeichnet, daß es zusätzlich Vitamin E bevorzugt in der Form von α-Tocopherolacetat enthält.

3. Arzneimittel nach Anspruch 1 oder 2, dadurch gekennzeichnet, daß die pharmakologisch brauchbare Magnesiumverbindung ein Magnesium-Salz mit einem pharmakologisch verträglichen Anion, wie Hydrogenphosphat, Glutamin, L-Hydrogenglutamat und/oder Orotat ist.

4. Arzneimittel nach einem der Ansprüche 1 bis 3, dadurch gekennzeichnet, daß es zusätzlich einen oder mehrere der folgenden Bestandteile Zinkoxid, Methionin und/oder ein Mucolyticum, wie Ambroxol.HCl oder N-Acetyl-L-cystein enthält.

5. Arzneimittel nach einem der Ansprüche 1 bis 4, dadurch gekennzeichnet, daß es in einer Dosiseinheitsform vorliegt, die bis zu 50 000 I.E. und bevorzugt etwa 22 500 I.E. Vitamin A, insbesondere bis zu etwa 200 mg Retinolpalmitat enthält.

6. Arzneimittel nach Anspruch 5, dadurch gekennzeichnet, daß es etwa 100 bis 200 mg Etofyllin, etwa 50 mg bis über 200 mg Vitamin C und die Magnesiumverbindung in einer Menge von bis zu 10 mg Magnesium pro Kilogramm Körpergewicht des zu behandelnden Patienten und gegebenenfalls einen oder mehrere der folgenden Bestandteile enthält: 30 mg bis etwa 100 mg α-Tocopherolacetat (Vitamin E), 1-3 mg Zinkoxid, 40-80 mg Methionin und eine wirksame Menge eines Mocolyticums.

7. Arzneimittel nach einem der Ansprüche 1 bis 6 in Dosiseinheitsform, dadurch gekennzeichnet, daß es enthält: 22 500 I.E. Vitamin A als Retinolpalmitat, 30 mg α-Tocopherolacetat, 150 mg Etofyllin, 250 mg Magnesiumhydrogenphosphat, 250 mg Magnesiumglutaminat und 50 mg Vitamin C, neben gegebenenfalls 2,5 mg Zinkoxid und/oder gegebenenfalls üblichen Hilfs- und Trägerstoffen.

8. Arzneimittel nach einem der Ansprüche 1 bis 7 in zur oralen Verabreichung, insbesondere in Drageeform, oder in zur parenteralen Verabreichung geeigneter Form.

9. Arzneimittel nach einem der vorliegenden Ansprüche zur Behandlung von entzündlichen Veränderungen der Bronchialschleimhaut.

Hans-Peter Walter                    M/WAL-11-EP
8022 Grünwald


P a t e n t a n s p r ü c h e
für den Vertragsstaat AT

1. Verfahren zur Herstellung eines Arzneimittels, dadurch gekennzeichnet, daß man Etofyllin, Vitamin A, eine oder mehrere pharmakologisch brauchbare Magnesiumverbindungen und Vitamin C, gegebenenfalls mit üblichen Hilfs- und Trägerstoffen, in üblicher Weise formuliert.

2. Verfahren nach Anspruch 1, dadurch gekennzeichnet, daß man zusätzlich Vitamin E bevorzugt in der Form von α-Tocopherolacetat verwendet.

3. Verfahren nach Anspruch 1 oder 2, dadurch gekennzeichnet, daß die pharmakologisch brauchbare Magnesiumverbindung ein Magnesium-Salz mit einem pharmakologisch verträglichen Anion, wie Hydrogenphosphat, Glutamin, L-Hydrogenglutamat und/oder Orotat ist.

4. Verfahren nach einem der Ansprüche 1 bis 3, dadurch gekennzeichnet, daß man zusätzlich einen oder mehrere der folgenden Bestandteile Zinkoxid, Methionin und/oder ein Mucolyticum, wie Ambroxol.HCl oder N-Acetyl-L-cystein verwendet.

5. Verfahren nach einem der Ansprüche 1 bis 4, dadurch gekennzeichnet, daß man eine Dosiseinheitsform formuliert, die bis zu 50 000 I.E. und bevorzugt etwa 22 500 I.E. Vitamin A, insbesondere bis zu etwa 200 mg Retinolpalmitat enthält.

6. Verfahren nach Anspruch 5, dadurch gekennzeichnet, daß man etwa 100 bis 200 mg Etofyllin, etwa 50 mg bis über 200 mg Vitamin C und die Magnesiumverbindung in einer Menge von bis zu 10 mg Magnesium pro Kilogramm Körpergewicht des zu behandelnden Patienten und gegebenenfalls einen oder mehrere der folgenden Bestandteile formuliert: 30 mg bis etwa 100 mg α-Tocopherolacetat (Vitamin E), 1-3 mg Zinkoxid, 40-80 mg Methionin und eine wirksame Menge eines Mocolyticums.

7. Verfahren nach einem der Ansprüche 1 bis 6 in Dosiseinheitsform, dadurch gekennzeichnet, daß man 22 500 I.E. Vitamin A als Retinolpalmitat, 30 mg α-Tocopherolacetat, 150 mg Etofyllin, 250 mg Magnesiumhydrogenphosphat, 250 mg Magnesiumglutaminat und 50 mg Vitamin C, mit gegebenenfalls 2,5 mg Zinkoxid und/oder gegebenenfalls üblichen Hilfs- und Trägerstoffen formuliert.

8. Verfahren nach einem der Ansprüche 1 bis 7, dadurch gekennzeichnet, daß man das Arzneimittel in zur oralen Verabreichung, insbesondere in Drageeform, oder in zur parenteralen Verabreichung geeigneter Form formuliert.

9. Verfahren nach einem der vorliegenden Ansprüche, dadurch gekennzeichnet, daß man das Arzneimittel zur Behandlung von entzündlichen Veränderungen der Bronchialschleimhaut herstellt.

**0121115**

Nummer der Anmeldung

**Europäisches Patentamt**

**EUROPÄISCHER RECHERCHENBERICHT**

EP 84 10 2238

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | UNLISTED DRUGS, Band 19, Nr. 5, Mai 1967, Seite 61p, Chatham, New Jersey, USA; * Seite 61p "SYMFONA" * --- | 1-9 | A 61 K 33/42<br>A 61 K 33/06<br>A 61 K 31/52 //<br>(A 61 K 33/42<br>A 61 K 31/52<br>A 61 K 31/375<br>A 61 K 31/07 ) |
| A | FR-M- 4 287 (HENRI BRUNEL) * Seite 1, rechte Spalte, Zusammenfassung * ----- | 1-9 | (A 61 K 33/06<br>A 61 K 31/52<br>A 61 K 31/375<br>A 61 K 31/07 )<br>(A 61 K 31/52<br>A 61 K 31/375<br>A 61 K 31/195<br>A 61 K 31/57 ) |

|  |  |  | RECHERCHIERTE SACHGEBIETE (Int. Cl. ³) |
|---|---|---|---|
|  |  |  | A 61 K |

Der vorliegende Recherchenbericht wurde für alle Patentansprüche erstellt.

| Recherchenort<br>DEN HAAG | Abschlußdatum der Recherche<br>12-06-1984 | Prüfer<br>BRINKMANN C. |
|---|---|---|